# EUROPEAN PATENT SPECIFICATION

(11) **EP 2 675 430 B1**
(45) Date of publication and mention of the grant of the patent: **10.10.2018**
(21) Application number: 12747704.0
(22) Date of filing: 14.02.2012
(51) Int. Cl.: A61K 8/64, A61K 8/34, A61Q 5/00, A61K 8/9706

(54) **A HAIR CARE PRODUCT AND A METHOD OF USING SAID PRODUCT**
HAARPFLEGEPRODUKT UND VERFAHREN ZUR VERWENDUNG EINES SOLCHEN PRODUKTS
PRODUIT DE SOIN CAPILLAIRE ET PROCÉDÉ D'UTILISATION DUDIT PRODUIT

(30) Priority: 18.02.2011 SE 1150134
(43) Date of publication of application: 25.12.2013
(73) Proprietor: Wide Vistas Entertainment AB, 129 40 Hägersten (SE)
(72) Inventor: OLOFSSON, Fredrik, S-129 40 Hägersten (SE)
(74) Representative: Ehrner & Delmar Patentbyrå AB
(86) International application number: PCT/SE2012/050145
(87) International publication number: WO 2012/112109

(56) References cited:
- EP-A1- 2 123 329
- JP-A- 1 313 415
- JP-A- 2000 290 143
- JP-A- 2003 300 843
- JP-A- 2004 143 091
- JP-A- 2005 314 359
- JP-A- 2009 067 719
- JP-A- 2009 263 302
- US-A1- 2006 286 058
- US-A1- 2011 311 655
- SEPPIC: 'Hair care formulary, nourishing and moisturizing hair care' COSMETICS & TOILETRIES vol. 120, no. 11, 2005, pages 2 - 7, XP002364662

## Description

### Field of the Invention

The present invention relates to a hair care product, according to claims 1 and 2, especially a product intended for the protection and care of long hair when shampooing it, as well as a method of using said product.

### Background

Many people dream of a long and healthy hair. The length of the growing phase and how long the hair becomes, are decided by our genes, and as a mean value a hair grows during seven years before it falls off. External influence such as styling with for example a blow-dryer or flat-irons can bring about heavy wear and the hair therefore need to be cut more often in order to look healthy and obtain a soft feeling. By using heat protecting products and lower heat this wear of the hair can be reduced to a certain extent, but what is mostly forgotten is that daily shampooing, despite all nourishing and re-moisturizing species in professional shampoos, also contributes to the drying-out, cleavage or even ripping off of the top. A hair having grown to a length of about 80 cm and having been washed every third day has been subjected to a shampoo at least 1 000 times at the top.

Already now there is a tendency that women look for an alternative to shampoo, when they experience that the cleaning wears the hair down, and instead they start to wash their hair with balm only (the old trick that like dissolves like, fat dissolves fat). At the beginning, one does not recognize the feeling of no lather, and this can be a less nice experience, since the skin on the scalp still overproduces fat, but gradually the production of tallow is restored, and balm is felt as a complete alternative to shampoo. The problem with this solution is that balm on the scalp in most cases does not give the same lightness and volume as a cleaning shampoo does.

There is also a dislike of choosing protein based hair care products, despite that the protein is what really strengthens the hair. Often the user does not notice an immediate improvement and rather chooses products with more fat making the hair glossy and soft, but, however, only cosmetically.

### The Invention

One object of the present invention is to provide a product according to claims 1 and 2, which solves or at least ameliorates the above noted problems in that it allows the user to continue to use a shampoo for cleaning while at the same time it protects the hair from the wear incurred by the cleaning.

This and other objects are achieved with the product according to the present invention with the characterizing features of claim 1. Further developments and preferred embodiments of the invention are defined in the sub claims.

The product according to claims 1 and 2 of the invention is a supplement to shampoo and balm and protects the lengths of the hair. It is a supplement to the daily hair care which spares the lengths from the wear of the shampoos. With this product in a hair care regime the user obtains it all, i.e. clean hair, filled with protein and gloss.

The invention also relates to a method of use of the product according to claims 1 and 2 of the invention in that it is applied in wet hair, on the lengths, before starting to shampoo, whereupon the hair is subsequently shampooed in the usual way, but only or mainly at the scalp. Thereafter both products are rinsed away and balm is applied as usual. The hair care product according to the invention, sometimes called primer, will protect the lengths from shampoo at rinsing, something that would not be the case when using only shampoo. The lengths will of course not be cleansed deeply in the same way, but the lengths do not have the same needs either, since fat is accumulated closest to the scalp. Simultaneously, however, the primer will still dissolve not too heavy dirt.

The product formula according to claims 1 and 2 of the invention will simply comprise the positive or nourishing properties of a shampoo for hair, but all reacting ingredients are excluded. The ingredients of the product counteract the washing effect and thereby also the lathering. A low pH value makes the hair to be sealed, which prevents the surface active agents of the shampoo from penetrating into the depth of the hair. The voids in the hair will be filled with protein. Moreover ingredients are added in order to obtain a higher surface tension in the water.

The product according to the invention will on the one hand quickly build up an impaired hair, on the other hand it prevents the hair from being damaged by washing. Simultaneously the hair closest to the scalp will receive the cleaning which is necessary to easily obtain the desired volume.

Further objects, advantages and characteristics of the invention will be evident from the following detailed description.

### Detailed description of the Invention

The invention is a hair care product according to claims 1 and 2 intended to be used by people with long hair, especially in connexion with shampooing, but, as will be evident from the following description, it can also be used in other situations in order to protect the lengths of the hair.

One important property of the product according to claims 1 and 2 of the invention is that it has only protecting properties and accordingly does not have any cleansing effect. To the contrary, the product should render it difficult for active detergent substances to get into contact with the lengths of the hair.

The hair care product according to claims 1 and 2 of the invention is a product which is used before shampooing but possible also, as will be evident from below, for protecting the hair in connection with other activities, comprises
- a substance with moisturizing and softening properties,
- an anti-static binding agent, which is film-forming, regulates the viscosity, and is a surfactant,
- a conditioning agent,
- an anti-static, softening and antifoam agent
- a film-forming, caring, and resealing agent,
- a pH controlling agent,
- the rest being water and possible additives, such as perfume, essential oils, etc.

One agent might fulfil several of the above functions.

The hair care product might contain optional other agents, such as perfumes, other fragrant substances, essential oils. Preservatives might occur and do not affect the action of the product.

More specifically, the hair care product according to the invention consists of
- sorbitol, as a moisture preserving and a softening agent, in an amount of from 4 to 12 % by weight;
- a thickener, such as guar flour, cellulose containing substances and/or gum arabicum, acting as an anti-static, film forming and surfactant agent, and as a conditioning agent, in an amount of from 1 to 5 % by weight,
- silk protein or a degraded product thereof as an anti-static, softening and antifoam agent in an amount of from 0,5 to 8 % by weight,
- a film-forming, caring and re-moisturizing agent such as algae extract in an amount of from 1 to 2 % by weight,
- a pH controlling and re-sealing agent, such as citric acid, vinegar, or similar, in an amount which gives the product a pH of about 4.4,
- optional additives, such as perfume, essential oils and similar, and
- remaining amount being water.

According to a more specific embodiment of the invention, the hair care product according to the invention consists of:

| | |
|---|---|
| sorbitol | 5 - 9 |
| a thickener | 1 - 3 |
| silk protein or | |
| a degraded product thereof | 0.5 - 4 |
| a re-moisturizing/caring substance | 1 - 2 |
| a pH controlling agent such as | |
| citric acid, vinegar or similar | to pH about 4.4 |

the rest up to 100 % is comprised of water and optional further additives, such as perfume, essential oils and similar, and optionally a preservative.

Guar flour in the form of hydroxy propyl guar can be used as anti-static, film forming, viscosity controlling and surfactant agent, in combination with hydroxypropyl guar-hydroxyl propyl trimonium chloride as conditioning and viscosity controlling agent. Alternative thickeners are for example cellulose based material, such as hydroxyetylcellulose, and gum arabicum.

The silk protein might be cocodimonium hydroxyl propyl hydrolyzed silk. Further, a degraded silk protein can be used, such as silk amino acids. Silk amino acids penetrates into the hair an add moisture, and also improves the plasticity of the hair, and make it less prone to breaking. Quaternary silk amino acids will attach more easy to the surface of the hair compared to not quaternized silk amino acids, they are even more conditioning, and they are effective at very low concentrations.

The algae extract might be hydrolyzed rodophycea extract, possibly in combination with chondrus crispus extract, and/or oligogeline (carrageenan) might be used. A further caring agent is Limnanthes Alba Seed oil.

The product according to claims 1 and 2 of the invention will obstruct the cleaning effect of surfactants. It accomplishes that the hair is sealed (the scale layer is sealed) because of a low pH value, while at the same time is gives gloss, moisturizes, and adds protein.

The product according to claims 1 and 2 should only be used in the lengths out to the top, starting at a distance from the scalp, and it will attach to the hair lengths as a protective cover/film/cream, before washing the hair. The product shall remain during shampooing and is intended to be used in connection with hair washing

The product according to claims 1 and 2 is suitable for long natural hair, chemically treated hair, and also for people with false hair/hair extensions and also people having scalp problems. Furthermore, the product will act excellently as an extra protection under a bathing cap in order to prevent chlorine water from entering into the hair.

The product according to claims 1 and 2 spares effectively the hair lengths when using dandruff shampoo, which is very deep-cleansing and drying-out.

People being subject to lice in the scalp often wash the hair so that the individual hairs are destroyed, since agents against lice are very strong. The product gives a good protection against this effect too in the lengths.

The invention is further illustrated in the following with specific examples of specific compositions. It should however be noted that they are given only as illustrative examples, not limiting the scope to the invention. The contents of the different ingredients are expressed in % by weight.

### Exemple 1

| | |
|---|---|
| Mackernium 261 (Guar Hydroxy propyl trimonium Chloride) ¹ | 1,9 |
| C*Sorbidex NC 16207 (Sorbitol)² | 7 |
| Crosilkquat (Cocodimonium Hydroxypropyl Silk Amino Acids)³ | 3,1 |
| Oligogeline (Carrageenan)² | 1,8 |
| Citric Acid | 0,05 |
| Euxyl PE 9010 (Phenoxyethanol, Ethylhexylglycerine)⁴ | 0,4 |
| Perfume Longanfruit 44756 (Perfume)⁵ | 0,04 |

Remaining part up to 100 % is water and unavoidable impurities.

### Exemple 2

| | |
|---|---|
| Mackernium 261 (Guar Hydroxypropyltrimonium Chloride)¹ | 1,9 |
| C*Sorbidex NC 16207 (Sorbitol)² | 5 |
| Crosilkquat (Cocodimonium hydroxypropyl Silk Amino Acids)³ | 3,1 |
| Oligogeline (Carrageenan)² | 1,8 |
| Citric Acid | 0,05 |
| Euxyl PE 9010 (Phenoxyethanol, Ethylhexylglycerine)⁴ | 0,4 |
| Perfume Longanfruit 44756 (Perfume)⁵ | 0,04 |

Remaining part up to 100 % is water and unavoidable impurities.
Suppliers: ¹ Vendico; ² Brenntag ; ³ Croda; ⁴ Bionord; ⁵ Kavat Kosmetik.

### Example 3

| | |
|---|---|
| Sorbitol | 7,19 |
| Crosilkquat LQ | 3,04 |
| Cosmedia Guar C 261 N | 1,49 |
| Natrosol 250 HHR | 0,35 |
| Oligogeline PF | 0,300 |
| Meadofoam Seed Oil | 2,45 |
| Citric Acid mono fine | 0,23 |
| Euxyl K-712 | 1,000 |
| Perfum Intensive Care | 0,18 |
| Water | up to 100 % |

### The Method according to the Invention

There are several different ways of washing hair:

### Normal wash with shampoo and balm:

- Wet the hair
- Add tensides/sulphates and massage up a lather/foam
- Rinse
- Add balm
- Rinse

In this case, all natural fats in the hair are washed away, said natural fats being the best protection for the hair, and adds artificial substances for the hair not to get entangled.

### The balm method:

- Wet the hair
- add balm and massage the scalp and the hair
- Rinse

A person having very dry hair can choose to wash the hair only with balm, according to the thesis like dissolves like, fat dissolves fat. On a thick, dry, naturally curly hair, this might well be a good solution, but for those having flat hair, the lift/feeling of clean hair will not be sufficient at the scalp, and will therefore fall flat.

### Method according to the Invention

- Rinse /soak the hair (here the water will dissolve light dirt, dust and particles, and water soluble hair styling products),
- Apply the hair care product according to the invention ("the primer") on the length of a distance from the scalp all the way to the top,
- Wash the scalp while avoiding to draw the shampoo out onto the primer.
- Rinse all of the hair, whereby the water and shampoo will bring the primer with them. Now the scalp is clean and the primer having been sitting in the hair for a while has had time to neutralize odours, add proteins and seal the individual hairs.
- Finish with balm for extra moisture and disentanglement.

Accordingly, in this case only the scalp is washed, the lengths are protected from tensides and sulphates, the proteins will enter into the lengths of the hair which strengthens the hair, the scale layer is sealed, and the procedure is finished with balm/disentanglement and extra gloss.

With the method according to the invention, the top/length reaching out longer than about 10 cm is not washed. The human hair grows about 8 - 10 cm in a year. This is about as long the hair can take daily shampooing without becoming split, cracked and get a dry feeling. Hair, which is older that about 1 year, will accordingly not be washed. The intention is that the length will rest from tensides as long as it is growing.

The method according to the invention is suitable also for those who uses extensions of the hair, since natural fat, which is the best lubricant for the hair, seldom can reach past the knots, where the hair is fastened. These lengths will therefore not obtain a natural lubrication, and will therefore dry out extremely fast. With the product according to the invention the lifetime can be extended also for false hair.

The product according to the invention is thus only applied onto the lengths and not on the skin of the scalp, and it protects and takes care of the hair.

The same washing effect will be experienced in the scalp and the hair closest to the scalp, but the length will be completely protected from washing.

It should be specifically pointed out that contrary to known products, the hair care product according to the invention shall not be rinsed away before shampooing.

If someone is staying in a dirtier environment than usual, and therefore feels a desire to wash also the lengths of hair, the shampoo is drawn out onto the product according to the invention sitting on the lengths of the hair, and the hair will then receive a gentler cleaning than if the product was not used. The low pH value makes it more difficult for the tensides to work, when they reach the protective layer on the separate hairs, with which is accomplished that the cleaning will be milder than if only shampoo is used.

An added value is that the shampoo consumption will be reduced, and it will be at least halved, since now only the scalp is to be washed, while at the same time the environmental load will be reduced, since the product does not contain tensides, and thanks to reduced use of shampoo the amount of tensides let out will be correspondingly reduced.

There is also the thought that optionally prevalent fragrances are only intended to neutralize non-wanted odours in the hair, such as smoke, perspiration, or alternatively in order to match the shampoo and balm, with which the product is launched.

## Claims

1. A hair care product to be used for protection and care of lengths of long hair when shampooing, **characterized in that** it consists of the following constituents:
- sorbitol, being a moisture preserving and a softening agent, in an amount of from 4 to 12 % by weight;
- a thickener selected from the group consisting of guar flour, cellulose containing substances and gum arabicum or a mixture thereof, acting as an anti-static, film forming and surfactant agent, and as a conditioning agent, in an amount of from 1 to 5 % by weight,
- silk protein and/or a degraded product therefrom, being an anti-static, softening and antifoam agent, in an amount of from 0.5 to 8 % by weight,
- an algae extract, being a film-forming, caring and re-moisturizing agent, in an amount of from 1 to 2 % by weight,
- a pH controlling and re-sealing agent selected from the group consisting of citric acid and vinegar, in an amount which gives the product a pH of about 4.4,
- optional additives, such as perfume, essential oils and similar, and
- remaining amount being water.

2. The hair care product according to claim 1, wherein the product is comprised of, in % by weight:
| | |
|---|---|
| sorbitol | 5 - 9 |
| a thickener selected from the group consisting of guar flour, cellulose containing substances and gum arabicum or a mixture thereof | 1 - 3 |
| silk protein, and/or | |
| a degraded product thereof | 0.5 - 4 |
| an algae extract | 1 - 2 |
| a pH controlling agent selected from the group consisting of citric acid and vinegar to pH about | 4.4, |
- optional additives, such as perfume, essential oils and similar,
- optionally a preservative, and
- remaining amount up to 100% being water.

3. A method for the protection and care of lengths of long hair when shampooing it with a product according to any of claim 1 or 2, **characterized in that** it comprises the following steps of:
i) rinsing/soaking the hair,
ii) applying the product according to claim 1 or 2 on the lengths about 10 cm from the scalp and all the way out towards the top,
iii) washing the scalp while avoiding drawing shampoo out over the hair lengths treated in step ii),
iv) rinsing all of the hair, whereby the water and shampoo will bring with them the product sitting on the hair lengths, resulting **in that** the scalp is clean and the product being present during the shampooing having had time to neutralize odours, add proteins and seal each hair, and
v) optionally, finishing with balm for extra moisture and disentanglement.

## Patentansprüche

1. Haarpflegemittel, das für Schutz und Pflege von Längen von langem Haar beim Shampoonieren zu verwenden ist, **dadurch gekennzeichnet, dass** es sich aus den folgenden Bestandteilen zusammensetzt:
- Sorbitol, das ein Feuchtigkeit erhaltendes und weichmachendes Mittel ist, in einer Menge von 4 bis 12 Gew.-%,
- einem Verdickungsmittel, das aus der Gruppe ausgewählt ist, die aus Guarkernmehl, zelluosehaltigen Substanzen und Gummi arabicum oder einer Mischung davon besteht, und das als ein antistatisches, filmbildendes und oberflächenaktives Mittel und als ein Konditionierungsmittel wirkt, in einer Menge von 1 bis 5 Gew.-%,
- Seidenprotein und/oder einem davon abgebauten Produkt, das ein antistatisches, weichmachendes und entschäumendes Mittel ist, in einer Menge von 0,5 bis 8 Gew.-%,
- einem Algenextrakt, der ein filmbildendes, pflegendes und rückbefeuchtendes Mittel ist, in einer Menge von 1 bis 2 Gew.-%,
- einem pH-regelnden und wieder abdichtenden Mittel, das aus der Gruppe ausgewählt ist, die aus Zitronensäure und Essig besteht, in einer Menge, die dem Produkt einen pH-Wert von ungefähr 4,4 gibt,
- wahlweisen Zusatzmitteln wie Parfüm, etherischen Ölen und ähnlichem und
- Restmenge, die Wasser ist.

2. Haarpflegemittel nach Anspruch 1, wobei das Produkt in Gew.-% aus Folgendem besteht:
Sorbitol 5 - 9
einem Verdickungsmittel, das aus der Gruppe ausgewählt ist, die aus Guarkernmehl, zellulosehaltigen Substanzen und Gummi arabicum oder einer Mischung davon besteht 1 - 3
Seidenprotein und/oder einem davon abgebauten Produkt 0,5 - 4
einem Algenextrakt 1 - 2
einem den pH-Wert regelnden Mittel, das aus der Gruppe ausgewählt ist, die aus Zitronensäure und Essig besteht, auf einen pH-Wert von ungefähr 4,4;
- wahlweisen Zusätzen, wie Parfüm, ätherischen Ölen und dergleichen,
- wahlweise einem Konservierungsstoff und
- Restmenge, die bis zu 100 % Wasser ist.

3. Verfahren für Schutz und Pflege von Längen von langem Haar beim Shampoonieren des Haars mit einem Produkt nach einem der Ansprüche 1 oder 2, **dadurch gekennzeichnet, dass** es die folgenden Schritte umfasst:
i) Spülen, Einweichen des Haars,
ii) Auftragen des Produkts nach Anspruch 1 oder 2 auf die Längen ungefähr 10 cm ab der Kopfhaut und den ganzen Weg nach oben,
iii) Waschen der Kopfhaut, und dabei verhindern, dass Shampoo über die in Schritt ii) behandelten Haarlängen gezogen wird,
iv) Ausspülen des gesamten Haars, wodurch Wasser und Shampoo das auf den Haarlängen sitzende Produkt mit sich bringen, mit dem Ergebnis, dass die Kopfhaut sauber ist und das Produkt, das während des Shampoonierens anwesend war, Zeit hatte, Gerüche zu neutralisieren, Proteine zuzufügen und jedes Haar abzudichten, und
v) wahlweise abschließende Behandlung mit einem Balsam für zusätzliche Feuchtigkeit und Entwirrung.

## Revendications

1. Produit de soin des cheveux destiné à être utilisé pour la protection et de soin des longueurs de cheveux longs lors d'un shampooing, **caractérisé en ce qu'**il est constitué des constituants suivants :
- du sorbitol, comme agent de conservation de l'hydratation et adoucissant, selon une quantité allant de 4 à 12% en poids ;
- un épaississant choisi dans le groupe constitué par la farine de guar, les substances à base de cellulose et de la gomme arabique ou un mélange de celles-ci, ayant un rôle d'antistatique, d'agent filmogène et tensioactif, et comme agent conditionneur, selon une quantité allant de 1 à 5% en poids,
- des protéines de soie et/ou un produit de dégradation de celles-ci, comme agent antistatique, conditionneur et antimousse, selon une quantité allant de 0,5 à 8% en poids,
- un extrait d'algues, comme agent filmogène, de soin et de réhydratation, selon une quantité allant de 1 à 2% en poids,
- un agent de contrôle de pH et de fermeture choisi dans le groupe constitué par l'acide citrique et le vinaigre, selon une quantité qui confère au produit un pH d'environ 4,4,
- des additifs facultatifs, tels qu'un parfum, des huiles essentielles et similaire, et
- la quantité restante étant constituée d'eau.

2. Produit de soin des cheveux selon la revendication 1, où le produit est constitué de, en % en poids :
- sorbitol 5-9
- un épaississant choisi dans le groupe constitué par la farine de guar, les substances à base de cellulose et de la gomme arabique ou un mélange de celles-ci 1-3
- des protéines de soie et/ou un produit de dégradation de celles-ci 0,5-4
- un extrait d'algues 1-2
- un agent de contrôle de pH choisi dans le groupe constitué par l'acide citrique et le vinaigre jusqu'à un pH d'environ 4,4,
- des additifs facultatifs, tels qu'un parfum, des huiles essentielles et similaire,
- éventuellement un conservateur, et
- la quantité restante jusqu'à 100% étant constituée d'eau.

3. Méthode de protection et de soin des longueurs de cheveux longs lors d'un lavage de ceux-ci avec un produit selon l'une quelconque des revendications 1 ou 2, **caractérisée en ce qu'**elle comprend les étapes suivantes consistant à :
i) rincer/tremper les cheveux,
ii) appliquer le produit selon la revendication 1 ou 2 sur les longueurs jusqu'à environ 10 cm du cuir chevelu et sur toute la longueur jusqu'aux pointes,
iii) laver le cuir chevelu tout en évitant de mettre du shampooing sur les longueurs de cheveux traitées dans l'étape ii),
iv) rincer l'ensemble de la chevelure, ce par quoi l'eau et le shampooing emmèneront avec eux le produit présent sur les longueurs de cheveux, faisant que le cuir chevelu sera propre et que le produit étant présent lors du shampooing aura eu le temps de neutraliser les odeurs, ajouter des protéines et refermer chaque cheveu, et
v) éventuellement, finir avec un baume pour hydrater davantage et démêler.
